# EUROPEAN PATENT APPLICATION

(11) **EP 1 197 229 A1**
(43) Date of publication of application: **17.04.2002**
(21) Application number: 00203560.8
(22) Date of filing: 13.10.2000
(51) Int. Cl.: A61L 2/00

(54) **Method of inactivating microorganisms**

(71) Applicant: Boston Clinics PDT B.V., 2333 AL Leiden (NL)
(72) Inventor: Dubbelman, Thomas Marinus Albert Remko, 2406 JJ Alphen a/d Rijn (NL); Lagerberg, Johannes Wilhelmus Maria, 2317 DV Leiden (NL); Trannoy, Laurence Liliane Annette, 2513 CG Den Haag (NL); Brand, Agatha, 1016 AE Amsterdam (NL)
(74) Representative: Kupecz, Arpad

(57) **Abstract**

The invention relates to a method of inactivating micro-organisms present in a liquid containing stem cells, where the method comprises the steps of
- combining said liquid with a photosensitiser, and
- activating the photosensitiser
In accordance with the present invention, a positively-charged sensitizer is used having a defined structure. Surprisingly, the use of these compounds minimize the damage to the stem cells.

## Description

The present invention relates to a method of inactivating micro-organisms present in a liquid containing stem cells comprising the steps of
- combining said liquid with a photosensitiser, and
- activating the photosensitiser.

Stem cells are more and more used to grow desired cell types, for example for transplantation purposes. A major source of stem cells is the umbilical cord. The solution containing the stem cells may be contaminated with various micro-organisms such as viruses (HIV, hepatitis B and C), fungi and bacteria. This severely limits or even rules out their use for the desired purpose.

It is known to inactivate organisms present in a blood product, such as plasma, red cells, platelets, leukocytes and bone marrow. For example, US 5,360,734 describes a method comprising the addition of the photosensitiser to the blood product and irradiation with light to activate the photosensitiser, thereby inactivating viral pathogens. Such a method causes damage to the cells, which is, for example, demonstrated by the hemolysis of erythrocytes. The method disclosed in US 5,360,734 is in particular aimed at and reducing the influence of plasma proteins in order to increase the stability of the red cells. Given the use of stem cells, damage to the cells is not acceptable.

The object of the present invention is to provide a method of inactivating micro-organisms in a liquid containing stem cells, minimizing the damage to the stem cells.

Thus, the present invention relates to a method according to the preamble characterized in that as the photosensitiser a compound is used chosen from the group consisting of compounds with the formulas Ia-Id 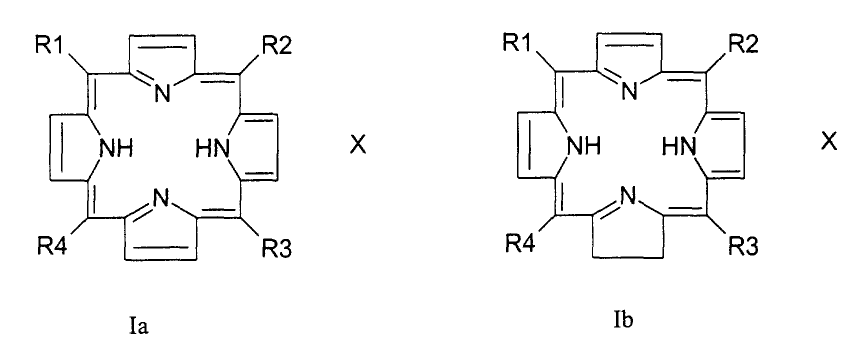 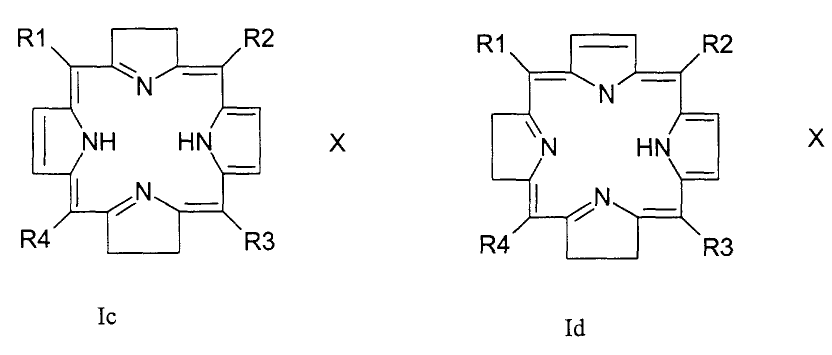 wherein R₁, R₂, R₃ and R₄ are independently chosen from the group consisting of
- hydrogen,
- a halogen atom,
- (C₁-C₂₀)alkyl, (C₁-C₂₀)alkoxy, (C₁-C₂₀)acyl, (C₁-C₂₀)acyloxy, (C₂-C₂₀)alkenyl, or (C₂-C₂₀)alkynyl, each of which may be linear or branched and each of which may be substituted with one or more groups chosen from
   - hydroxyl,
   - amino which may be substituted with 1 to 3 groups chosen from (C₁-C₂₀)alkyl, (C₂-C₂₀)alkenyl, (C₁-C₂₀)alkoxy, (C₂-C₂₀)alkynyl, and -(R₅-Z)ₘ-R₆ where R₅ is (CH₂)ₙ, Z is O or S, and R₆ is (C₁-C₂₀)alkyl and m and n are, independently, 1-10, each substituent group of the amino group may be linear or branched and each of these may be substituted with one or more groups chosen from hydroxyl, and a halogen atom,
   - nitril, and
   - a halogen atom,
- (C₆-C₂₀)aryl, and (C₆-C₂₀) heterocyclic aryl group each of which may be substituted with one or more groups chosen from
   - hydroxyl,
   - amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
   - nitril,
   - a halogen atom, and
   - (C₁-C₁₀)alkyl, (C₁-C₁₀)alkoxy, (C₂-C₁₀)alkenyl,
   the heterocyclic aryl group containing at least one atom chosen from N, O, P, and S where P, N or S may be substituted with a group chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
at least one of the groups R₁, R₂, R₃ and R₄ contains a quaternary nitrogen atom, and
wherein X is a pharmaceutically acceptable counterion.

Surprisingly, it has been found that by using a photosensitising compound as disclosed above, the damage to the stem cell substantially avoided, even if no protecting agent (such as disclosed in PCT/NL99/00387) is present

In the present invention, when referring to liquid containing stem cells, the term "liquid" is to be understood as any aqueous solution comprising at least stem cells. Other cell types may be present and the solution, while being an aqueous solution, may contain proteinaceous components, salts, stabilizers, as is generally recognized in the art.

The term "photosensitizer" is, as well recognized in the art, a substance which absorbs light energy as a result of which the photosensitizer is activated. The activated photosensitizer can subsequently react with other compounds. This may result in the photosensitizer being modified or inactivated, but more likely the photosensitizer will return to its original state (before it was activated with light), so as to form a photocatalytic cycle in which the photosensitizer can be used again. In effect, the light energy absorbed is used for the inactivation of micro-organisms.

The term "viral particle" is understood to mean any RNA or DNA virus, single- or double-stranded and with a membrane or proteinaceous coat that may occur in a stem cell-containing liquid. Examples are HIV and hepatitis B.

The term "pharmaceutically acceptable counterion" is understood to be any inorganic or organic negatively charged counterion such as OH⁻, Cl⁻, acetate or citrate. It goes without saying that there are as many counterions X as needed to neutralise the positive charge of the actual active compound I

According to a first embodiment at least one of R₁, R₂, R₃ and R₄ is a (C₆-C₂₀) heterocyclic aryl group comprising a nitrogen atom substituted with a group chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom. Preferably, the heterocyclic aryl group is a pyridinium group, the nitrogen of which is substituted with a (C₁-C₄) alkyl group.

According to a second embodiment at least one of R₁, R₂, R₃ and R₄ is a (C₆-C₂₀) aryl group substituted with an amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom. Preferably the aryl group is a trialkyl aminophenyl group where alkyl is independently (C₁-C₃) alkyl.

It has been found that quaternary nitrogen atoms in groups as defined above are very suitable for eliminating viral particles while maintaining the integrity of the stem cells.

Preferably at least two of R₁, R₂, R₃ and R₄ comprise a quaternary nitrogen atom, and more preferably three of R₁, R₂, R₃ and R₄ comprise a quaternary nitrogen atom. Such compounds appear to give the best result.

The invention will now be elucidated with reference to the exemplary embodiments and the drawing, in which
Fig. 1 shows the effective elimination of a bacterium from a stem cell-containing solution;
Fig. 2 shows the viability of stem cells before and after treatment; and
Fig. 3 represents a bar diagram to show the effect of the stem cell treatment in comparison with untreated cells.

### EXAMPLES

### Stem cells

Stem cells were isolated from fresh umbilical cord blood using hydroxy ethylstarch (HES) sedimentation (Perutelli P. et al, Vox Sang. 76(4) pp 237-40 (1999); Adorno G. et al, Clin. Lab. Haematol. 20(6) pp 341-3 (1998)). The resulting solution contained 20*10⁶ cells/ml, as determined using an automatic cell counter (Sysmex K1000, TOA Medical Electronics, Kobe, Japan).

### Photodynamic treatment of stem cell-containing solution contaminated with Pseudomonas

Stock solutions of Pseudomonas aeruginosa were added to the stem cell solution such that the volume of the spike was <10% of the total volume of the solution, and the number of bacteria was about 10⁵/ml. A sensitizer, monophenyl-tri(N-methyl-4-pyridyl)porphyrin chloride (TriP(4), Mid-Century, Posen, Illinois, USA), was added to a final concentration of 25 µM. The suspension were thoroughly mixed and divided into 3-ml aliquots in polystyrene culture dishes with a diameter of 6 cm (Greiner, Alphen a/d Rijn, the Netherlands), and agitated at room temperature on a horizontal reciprocal shaker (60 cycles/min, GFL, Burgwedel, Germany) for 5 min. in the dark. The dishes (one dish per time point) were illuminated from above with a 300 W halogen lamp (Philips, Eindhoven, the Netherlands). The light passed through a 1-cm water filter, to avoid heating of the samples. A cut-off filter, only transmitting light with wavelengths above 600 nm, was used in all experiments. The irradiance at the cell layer was 100 W/m², as measured with an IL1400A photometer equipped with a SEL033 detector (International Light, Newburyport, MA, USA). The following parameters were measured:
- Inactivation of P. aeruginosa; and
- Viability of the stem cells.
- Influence of the treatment on stem cell differentiation (as measured by cfu-e, bfu-e, cfu-m, cfu-gm, and cfu-gem).

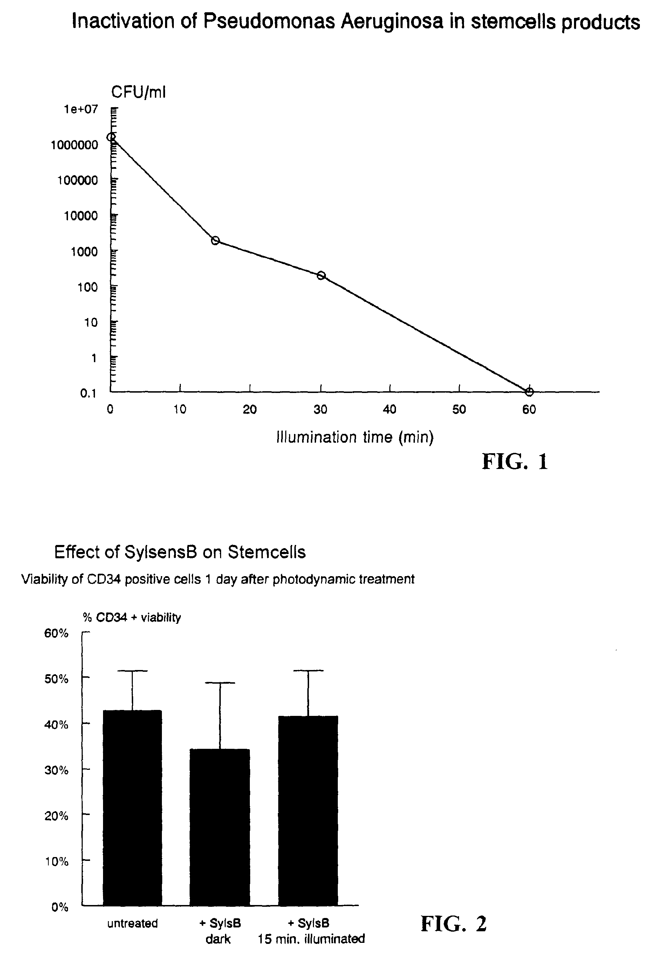

Fig. 1 shows that the bacteria were effectively killed during the treatment.

Fig. 2 shows that the viability of CD34-positive cells (stem cells) remains within the error margin for the control experiment (no illumination, no sensitizer).

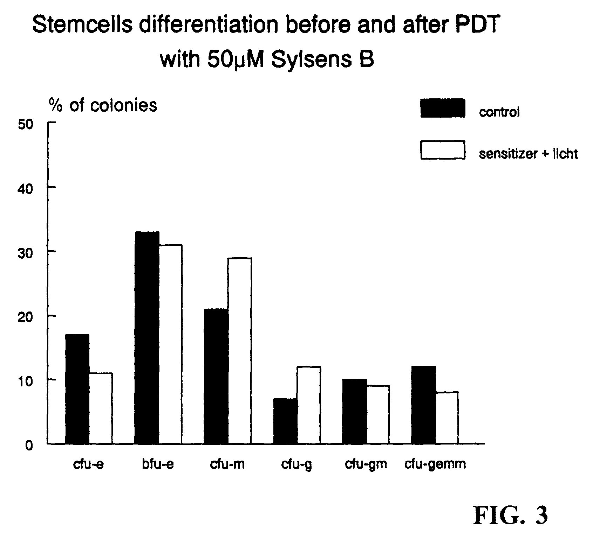

Fig. 3 shows the results of the various test on stem cell differentiation (determined as described in the MegaC-ult-C Technical Manual. Assays for Quantitation of Human and Murine Megakaryocytic Progenitors. version 3.0.3, March 1999, Stem Cell Technologies Inc., Vancouver, Canada). Please note that the data shown are from one experiment only, instead of, as usual, an average of several experiments. Bearing this in mind, these first results indicate that there is no significant effect of the treatment on the stem cell differentiation. For the experiments of fig. 2 and 3, the duration of the illumination was 1 hour.

## Claims

1. Method of inactivating micro-organisms present in a liquid containing stem cells comprising the steps of
- combining said liquid with a photosensitiser, and
- activating the photosensitiser,
**characterized, in that** as the photosensitiser a compound is used chosen from the group consisting of compounds with the formulas Ia-Id 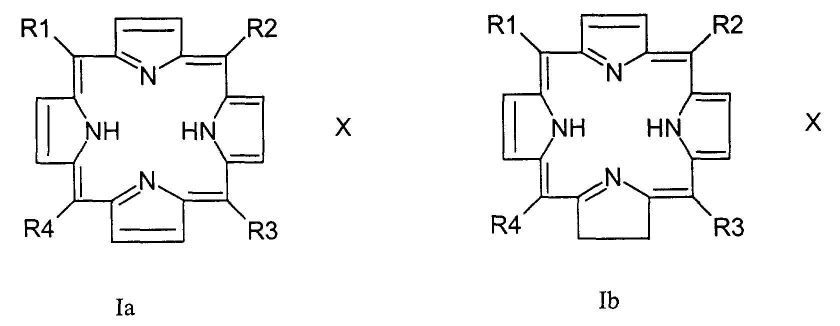 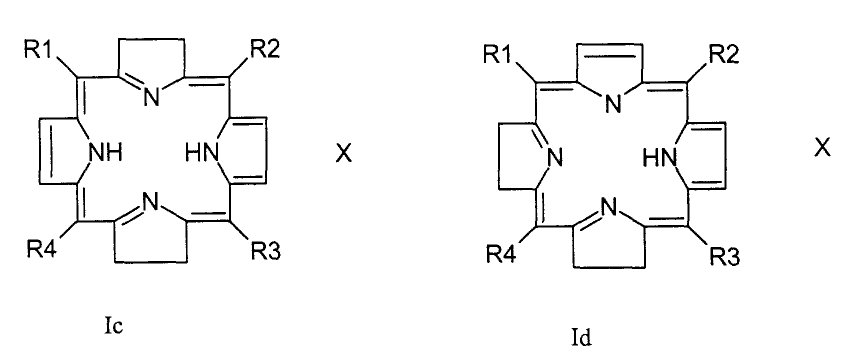 wherein R₁, R₂, R₃ and R₄ are independently chosen from the group consisting of
- hydrogen,
- a halogen atom,
- (C₁-C₂₀)alkyl, (C₁-C₂₀)alkoxy, (C₁-C₂₀)acyl, (C₁-C₂₀)acyloxy, (C₂-C₂₀)alkenyl, or (C₂-C₂₀)alkynyl, each of which may be linear or branched and each of which may be substituted with one or more groups chosen from
- hydroxyl,
- amino which may be substituted with 1 to 3 groups chosen from (C₁-C₂₀)alkyl, (C₂-C₂₀)alkenyl, (C₁-C₂₀)alkoxy, (C₂-C₂₀)alkynyl, and -(R₅-Z)ₘ-R₆ where R₅ is (CH₂)ₙ, Z is O or S, and R₆ is (C₁-C₂₀)alkyl and m and n are, independently, 1-10, each substituent group of the amino group may be linear or branched and each of these may be substituted with one or more groups chosen from hydroxyl, and a halogen atom,
- nitril, and
- a halogen atom,
- (C₆-C₂₀)aryl, and (C₆-C₂₀) heterocyclic aryl group each of which may be substituted with one or more groups chosen from
- hydroxyl,
- amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
- nitril,
- a halogen atom, and
- (C₁-C₁₀)alkyl, (C₁-C₁₀)alkoxy, (C₂-C₁₀) alkenyl,
the heterocyclic aryl group containing at least one atom chosen from N, O, P, and S where P, N or S may be substituted with a group chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom,
at least one of the groups R₁, R₂, R₃ and R₄ contains a quaternary nitrogen atom, and
wherein X is a pharmaceutically acceptable counterion.

2. Method according to claim 1, **characterized, in that** at least one of R₁, R₂, R₃ and R₄ is a (C₆-C₂₀) heterocyclic aryl group comprising a nitrogen atom substituted with a group chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom.

3. Method according to claim 2, **characterized, in that** the heterocyclic aryl group is a pyridinium group, the nitrogen of which is substituted with a (C₁-C₄) alkyl group.

4. Method according to claim 1, **characterized in that** at least one of R₁, R₂, R₃ and R₄ is a (C₆-C₂₀) aryl group substituted with an amino which may be substituted with 1 to 3 groups chosen from (C₁-C₂₀)alkyl, (C₂-C₂₀)alkenyl, (C₁-C₂₀)alkoxy, and (C₂-C₂₀)alkynyl, each of which may be linear or branched and each of which may be substituted with one or more groups chosen from hydroxyl, and a halogen atom.

5. Method according to claim 4, **characterized in that** the aryl group is a trialkyl aminophenyl group where alkyl is independently (C₁-C₃) alkyl.

6. Method according to any of the preceding claims, **characterized in that** at least two of R₁, R₂, R₃ and R₄ comprise a quaternary nitrogen atom.

7. Method according to claim 6, **characterized in that** three of R₁, R₂, R₃ and R₄ comprise a quaternary nitrogen atom.
